# EUROPEAN PATENT APPLICATION

(11) **EP 1 500 391 A1**
(43) Date of publication of application: **26.01.2005**
(21) Application number: 03291833.6
(22) Date of filing: 24.07.2003
(51) Int. Cl.: A61K 31/135, A61P 21/00, A61P 25/00, A61P 25/18, A61P 25/22, A61P 25/28

(54) **Therapeutic use of bicycloheptane derivatives**

(71) Applicant: NEURO3D, 68100 Mulhouse (FR)
(72) Inventor: Mondadori, Cesare, 4153 Reinach (CH); Paparin, Jean-Laurent, 67400 Illkirch (FR); Biondi, Stefano, 67000 Strasbourg (FR); Joseph, Christophe, 67000 Strasbourg (FR)
(74) Representative: Gallois, Valérie

(57) **Abstract**

The invention refers to bicycloheptane derivatives and their use in the treatment of autism, fibromyalgia, sleep disorders, bipolar disorder, mania, obsessive compulsive disorder, social phobia, anorexia, cognition disturbances, or age related disturbances.

## Description

### TECHNICAL FIELD OF THE INVENTION

The invention refers to (1R,2S,4R)-(-)-2-[N,N-(dimethylaminoethoxy)]-2-phenyl-1,7,7-trimethyl-bicyclo[2.2.1]heptane, also known as deramciclane, (1S,2R,4S)-(+)-2-[N,N-(dimethylaminoethoxy)]-2-phenyl-1,7,7-trimethyl-bicyclo[2.2.1]heptane, (1RS,2SR,4RS)-2-[N,N-(dimethylaminoethoxy)]-2-phenyl-1,7,7-trimethyl-bicyclo[2.2.1]heptane, one of their active metabolites or a mixture thereof, and their use in the treatment of autism, fibromyalgia, sleep disorders, bipolar disorder, mania, obsessive compulsive disorder, social phobia, anorexia, cognition disturbances, or age related disturbances.

### BACKGROUND OF THE INVENTION

It is known that such bicycloheptane derivatives have anticonvulsive, motility inhibiting and analgesic activities, furthermore potentiate the narcosis induced by hexobarbital. In some compounds, the above main activities are supplemented by weak antiserotonin, gastrointestinal peristaltic inhibiting and antiinflammatory effects (U.S. Pat. No. 4,342,762). U.S. Pat. No. 5,652,270 also describes that such compounds are suitable for the treatment of diseases and disorders connected with functionaldisturbances of the cholecystokinin (CCK) system. Moreover, deramciclane is also described to have anxiolytic-like and anti-depressant effects (WO 01/68067). In man, deramciclane undergoes biotransformation into N-desmethyl-deramciclane, which is an active metabolite for treating anxiety, as described in US 6,093,747.
These references are herein incorporated by reference in their entirety.

Age related disturbances include disturbances in the process of memorization, and retrieval, and a general decline in the ability to judge. A decline in memory and cognitive function (thinking) is often considered to be a normal consequence of aging. Age-related cognitive decline is therefore usually not considered a disease. However, mild cognitive impairment (MCI) has been considered a condition separate from normal aging and separate from Alzheimer's disease. More than 80% of patients with mild cognitive impairment develop Alzheimer's disease within 10 years at a rate of 10% to 15% of patients per year. Mild Cognitive Impairment (MCI) is a condition characterized by mild recent memory loss without dementia or significant impairment of other cognitive functions to an extent that is beyond that expected for age or educational background. The following criteria are assigned to MCI: memory complaint; normal activities of daily living; normal general cognitive functioning; abnormal memory for age; not demented.

Anorexia nervosa (AN) and bulimia are life-threatening disorders affecting mostly adolescent women. AN at least is a dramatic psychiatric syndrome accompanied by severe weight loss, hyperactivity and neuroendocrine changes. Several studies have shown a strong genetic component in AN.Several candidate genes have been screened for association with anorexia nervosa, including the 5HT2A receptor gene, the 5HT transporter gene, the dopamine D3 and D4 receptor gene, the 5HT1D and 5HT7 receptor genes, the leptin gene, the MC4-r gene, the POMC gene, NPY-Y5 receptor gene, the estrogen receptor beta and the beta 3-adrenergic-receptor. Moreover, weight gain in schizophrenia had been associated with the antagonistic properties of antipsychotic compounds on the 5HT2c receptor.

Social phobia is a common psychological problem which includes an unreasonable, marked and persistent fear of one or more social or performance situations in which a person is exposed to unfamiliar people or to possible scrutiny by others. Social phobia may be focused or generalized. In focused cases, the person usually fears a specific discrete setting, i.e., performance anxiety or stage fright. Generalized social phobia relates to a fear of most social situations. Because of the anticipatory nature of these events, anxiety can overwhelm the patient, rendering him unable to perform social obligations.

Cognitive processing by a person suffering from social phobia is characterized by a large amount of negative thoughts correlated to the levels of anxiety and phobic severity.

The victim of social phobia frequently experience physical symptoms characteristic of autonomic nervous system activation: dry mouth, blushing, palpitations, sweating, trembling, nausea and diarrhea. A social phobic attack may also include symptoms such as twitching and stammering.

Compared to patients with panic disorder, patients with social phobia tend to have a greater frequency of blushing, muscle twitching, stammering, and dry mouth, and a lower incidence of dizziness or respiratory symptoms.

Social phobia has been treated with drugs, behavioral therapy or a combination pharmaceutical and behavioral approach, with mixed results. There is no single drug approved for the treatment of social phobia. There is a need therefore to find a drug effective for the treatment of social phobia.

Obsessive-compulsive disorder (OCD), one of the anxiety disorders, is a potentially disabling condition that can persist throughout a person's life. The individual who suffers from OCD becomes trapped in a pattern of repetitive thoughts and behaviors that are senseless and distressing but extremely difficult to overcome. OCD occurs in a spectrum from mild to severe, but if severe and left untreated, can destroy a person's capacity to function at work, at school, or even in the home. A recent NIMH survey showed that OCD affects more than 2 percent of the population, meaning that OCD is more common than such severe mental illnesses as schizophrenia, bipolar disorder, or panic disorder. OCD strikes people of all ethnic groups. Males and females are equally affected. The obsessions are unwanted ideas or impulses that repeatedly well up in the mind of the person with OCD. Persistent fears that harm may come to self or a loved one, an unreasonable concern with becoming contaminated, or an excessive need to do things correctly or perfectly, are common. These thoughts are intrusive, unpleasant, and produce a high degree of anxiety. Sometimes the obsessions are of a violent or a sexual nature, or concern illness. The most frequent compulsions are washing and checking. Other compulsive behaviors include counting (often while performing another compulsive action such as hand washing), repeating, hoarding, and endlessly rearranging objects in an effort to keep them in precise alignment with each other. Mental problems, such as mentally repeating phrases, listmaking, or checking are also common. These behaviors generally are intended to ward off harm to the person with OCD or others. Some people with OCD have regimented rituals while others have rituals that are complex and changing. Performing rituals may give the person with OCD some relief from anxiety, but it is only temporary.

Treatments include behavioural therapy and some pharmacotherapy. Clinical trials in recent years have shown that drugs that serotonine uptake inhibitors do have positive effects on the symptoms of OCD. Clomipramine, and some selective serotonine uptake inhibitors have been approved for the indication. A polymorphism in the D4 gene has been proposed to be connected with OCD.

Sleep disorders are becoming increasingly prevalent in our society. It is estimated that 40 million Americans suffer from various sleep disorders. Further, 25 million more Americans suffer from intermittent-sleep-related disorders. Sleep disorders have various etiologies including stress induced by environmental and life style factors, physical factors, such as disease or obesity, and psychiatric disorders, such as depression.

Sleep disorders encompass, among other things, snoring, sleep apnea, insomnia, narcolepsy, restless legs syndrome, sleep terrors, sleep walking, sleep eating, disturbances in falling asleep, and disturbance of sleep architecture. Possible treatment can be as simple as behavior modification or it can be as involved as mechanical, surgical, or pharmacological intervention.

Sleep difficulties are a major motivation for use of drugs. The most often selected drugs are those interacting with the GABA neurotransmitter-receptor system in the brain, the so-called minor tranquillisers, of which the group of benzodiazepine drugs is the classic example. Disadvantages of currently available hypnotics are the potential for adverse reactions, remaining lesser quality of sleep, hangover effects, dependency potential, withdrawal effects and undesirable effects on cognitive functioning. The quality of sleep can not only be derived from the effect of sleep, for example whether the sleep has been refreshing and has a positive effect on daytime drowsiness/alertness the morning after, but also from objective EEG determined characteristics, describing sleep stages and architecture.

Autism is a disabling neurological disorder that affects millions of people and includes a number of subtypes, with various assumed causes and few documented ameliorative treatments. Autism is characterized by behavioral syndrome often recognized between two and three years of age. There is no clear-cut biological marker for autism. Diagnosis of the disorder is made by considering the degree to which the child matches the behavioral syndrome, which is characterized by poor communicative abilities, peculiarities in social and cognitive capacities, and maladaptive behavioral patterns.

There currently is no known medical treatment for autism. A number of different therapies have been attempted in an effort to cure autism or at least lessen its symptoms, including drug therapies as well as psychiatric care and attempted counseling. In general, results of such treatments have been disappointing, and autism remains very difficult to effectively treat, particularly in severe cases. There remains a need in the art for a pharmaceutical treatment offering clinical improvement in the autistic patient.

Fibromyalgia is a common disabling disorder characterized by chronic musculoskeletal aches and pain, stiffness, general fatigue, and sleep abnormalities including diminished stage four sleep. Fibromyalgia is a chronic, painful disorder commonly seen in rheumatology practice and is often viewed as a musculoskeletal pain process. Fibromyalgia is characterized as a reproducible, neurosensory processing abnormality associated with fatigue, and generalized muscular spasm, which most rheumatologists suspect is related to stage IV sleep deprivation. Examination of affected patients reveals increased tenderness at muscle and tendon insertion sites, known as "tender points". Fibromyalgia patients experience severe morning stiffness and a generalized decreased of overall physical function, and they are often prone to headaches, memory and concentration problems, dizziness, numbness and tingling, and crampy abdominal or pelvic pain. Fibromyalgia affects 2-4% of the population and is most frequently found in women between 20 and 50 years old, though it can also affect men, the elderly and minors.

Diagnosis of fibromyalgia is often overlooked due to the general nature of the symptoms and the lack of diagnostic lab or x-ray abnormalities. The disorder is often concomitant with, masked by or confused with other diseases such as rheumatoid arthritis, chronic fatigue syndrome or irritable bowl syndrome. A physician can positively diagnose fibromyalgia syndrome by finding the symptoms of generalized musculoskeletal pain and pain at more than 11 of 18 defined characteristic "tender points" when finger pressure is applied to the area. The total pain score for all 18 tender points is referred to as the "tender point index" of that patient.

The etiology of fibromyalgia is not known but consideration has been given to genetic, traumatic, affective, and infectious processes as possibilities. Currently the best treatment available for fibromyalgia consists of a combination of analgesics, sleep aids, exercise programs emphasizing stretching and cardiovascular fitness, relaxation techniques and other measures to reduce muscle tension, and educational and psychological support programs to reduce emotional and physical stress; the resulting benefits are usually disappointing. Numerous pharmaceutical regimes have been tried including treatment with serotonin modulators and antisera to endogenous psychoactive agents. Therapeutic response can be assessed by the reduction of pain in the tender point index and improvement in several generalized criteria such as physical function, stiffness, fatigue, depression, tenseness, etc. Responses to these various therapies have proven variable within a patient pool and have rarely exceeded modest relief of some symptoms.

There remains a need in the art for a pharmaceutical treatment offering clinical improvement in the patient suffering from such disorder.

Episodes of mania occur in patients who suffer from bipolar disorder which is an illness characterized by alternating cycles of depression and mania. Mania presents various forms whether acute or chronic, single or recurrent episode, and associated with depression or not. Episodes of acute mania are characterized by elevated or irritable mood, disturbed sleep, grandiosity, increased motor activity, pressured thinking, distractibility and poor concentration, impaired judgment, and sometimes psychotic symptoms. The irritability can lead to outbursts of angry or aggressive behavior. Often the episodes are preceded by a period of disturbed sleep. The distractibility makes the patient move endlessly from one activity to another often to the detriment of their physical, occupational, and social well-being. The impact of these behaviors is further aggravated by the lapses of judgment and poor decision-making that is characteristic of this illness.

Bipolar disorder is distinct from the more common form of depression, called Major Depressive Disorder, in which patients only experience recurrent episodes of depression but no mania. Bipolar disorder can be diagnosed by the clinical evaluation of patients using the criteria specified in the Diagnostic and Statistical Manual (DSM-IV) of the American Psychiatric Association. In this nomenclature system, bipolar disorder is subsumed under the broader class of Mood Disorders and is clearly distinguished from the Anxiety Disorders and from Organic Mental Disorders.

Bipolar Disorder is a psychiatric condition which is prevalant across cultures and age groups. Bipolar Disorder is a recurrent disorder characterized by one or more Manic Episodes immediately before or after a Major Depressive Episode or may be characterized by one or more Major Depressive Episodes accompanied by at least one Hypomanic Episode. Additionally, the symptoms must cause clinically significant distress or impairment in social, occupational, or other important areas of functioning. In some cases the Hypomanic Episodes themselves do not cause impairment; however, the impairment may result from the Major Depressive Episodes or from a chronic pattern of unpredictable mood episodes and fluctuating unreliable interpersonal and occupational functioning. The symptoms of Bipolar Disorder must not be better accounted for by a psychotic condition or due to the direct physiological effects of a medication, other somatic treatments for depression, drugs of abuse, or toxin exposure.

Bipolar Disorder is associated with a significant risk of completed suicide.

Therefore, Bipolar Disorder is a serious, fairly prevalent, psychological condition which is clearly distinguished from other psychotic conditions such as schizophrenia and depression.

### SUMMARY OF THE INVENTION

The present invention is based on the discovery that the compounds above mentioned present specific receptor binding profiles which combine activities on dopaminergic D3 and D2, 5HT2a, 5HT2c, 5HT6, and sigma receptors, plus Na channels systems.

The present invention is based on pharmacologal effects of the compounds described which indicate their usefulness for the treatment of a series of disorders. These disorders include age related disturbances, Anorexia Nervosa, social phobia, obsessive compulsive disorder, sleep disorders, autism, fibromyalgia, mania, and bipolar depression.

Indeed, the unique combination of anti-dopaminergic, and anti-serotonergic effects of the bicycloheptane derivatives might provide treatment not only to the cognition part of age-related disturbances but also for other age related mental disturbances such as confusion and agitation

Moreover, the combination of antagonism of 5HT2a and 5HT2c of the bicycloheptane derivatives is expected to be a pharmacotherapy for patients with AN.

Furthermore, the pharmacological profile of the bicycloheptane derivatives is also expected to have positive effects on social phobia.

On the basis of the combination of effects on serotonin and dopamine receptors of the bicycloheptane derivatives, the present invention provides a new way to treat obsessive compulsive disorder.

The unique combination of pharmacological features of the bicycloheptane derivatives will also provide a new way to treat sleep disturbances with a minimal risk of unwanted side effects, to treat autism or to treat fibromyalgia.

The unique combination of features of bicycloheptane derivatives especially the combination of effects on serotonergic, dopaminergic effects in combination with effects on the sodium channel. might provide a new way to treat mania and bipolar disorder.

It is an object of the invention to provide a method for treating autism, fibromyalgia, sleep disorders, bipolar disorder, mania, obsessive compulsive disorder, social phobia, anorexia, cognition disturbances, or age related disturbances, comprising administering an effective amount of bicycloheptane derivatives selected in the group consisting of (1R,2S,4R)-(-)-2-[N,N-(dimethylaminoethoxy)]-2-phenyl-1,7,7-trimethyl-bicyclo[2.2.1]heptane, (1S,2R,4S)-(+)-2-[N,N-(dimethylaminoethoxy)]-2-phenyl-1,7,7-trimethyl-bicyclo[2.2.1]heptane, (1RS,2SR,4RS)- 2-[N,N-(dimethylaminoethoxy)]-2-phenyl-1,7,7-trimethyl-bicyclo[2.2.1]heptane, one of their active metabolites, a mixture thereof, and a pharmaceutically acceptable salt thereof to a patient in need of such treatment. More preferably, said pharmaceutically acceptable salt is a fumarate salt.

Another object of the invention is to provide a use of bicycloheptane derivatives selected in the group consisting of (1R,2S,4R)-(-)-2-[N,N-(dimethylaminoethoxy)]-2-phenyl-1,7,7-trimethyl-bicyclo[2.2.1]heptane, (1S,2R,4S)-(+)-2-[N,N-(dimethylaminoethoxy)]-2-phenyl-1,7,7-trimethyl-bicyclo[2.2.1]heptane, (1RS,2SR,4RS)- 2-[N,N-(dimethylaminoethoxy)]-2-phenyl-1,7,7-trimethyl-bicyclo[2.2.1]heptane, one of their active metabolites, a mixture thereof, and a pharmaceutically acceptable salt thereof, for the manufacture of a medicament for therapeutic application in treating autism, fibromyalgia, sleep disorders, bipolar disorder, mania, obsessive compulsive disorder, social phobia, anorexia, cognition disturbances, or age related disturbances. More preferably, said pharmaceutically acceptable salt is a fumarate salt.

The active metabolites include preferably (1R,2S,4R)-(-)-2-methylaminoethoxy-2-phenyl-1,7,7-trimethyl-bicyclo[2.2.1]heptane, (1S,2R,4S)-(+)-2-methylaminoethoxy-2-phenyl-1,7,7-trimethyl-bicyclo[2.2.1]heptane, and (1RS,2SR,4RS)-2-methylaminoethoxy-2-phenyl-1,7,7-trimethyl-bicyclo[2.2.1]heptane.

The pharmaceutically acceptable salt of the above compounds includes any pharmaceutically acceptable acid addition salt or quaternary ammonium derivative thereof. The pharmaceutically acceptable acid addition salt can be formed with inorganic acids (e.g. hydrogen halides such as hydrochloric acid or hydrogen bromide or sulphuric acid, phosphoric acid or nitric acid, etc.) or organic acids (e.g. tartaric acid, succinic acid, malic acid, maleic acid, fumaric acid, citric acid, lactic acid, methanesulfonic acid or p-toluenesulfonic acid etc.). The quaternary ammonium derivatives of the above compounds can be formed by reacting the bicycloheptane derivatives with alkyl halides (e.g. methyl, ethyl, n-propyl or isopropyl chloride, bromide or iodide, etc.)

From the pharmaceutically acceptable acid addition salts the (E)-3-butenedioates formed with fumaric acid were found to be especially preferred.

### DETAILED DESCRIPTION OF THE INVENTION

This method includes, but is not limited to the treatment of mania in all its various forms whether acute or chronic, single or recurrent episode, and associated with depression or not.

The invention further includes the preventive treatment of bipolar disorder in persons predisposed to this disorder.

The presently claimed invention provides a method for treating bipolar disorder, comprising administering an effective amount of one of bicycloheptane derivatives as defined above or a pharmaceutically acceptable salt thereof to a patient in need of such treatment. More preferably, said pharmaceutically acceptable salt is a fumarate salt.

The present invention provides a method for treating depressive/depressive bipolar disorder, comprising administering an effective amount of one of bicycloheptane derivatives as defined above or a pharmaceutically acceptable salt thereof to a patient in need of such treatment. More preferably, said pharmaceutically acceptable salt is a fumarate salt.

Generally, Bipolar Disorder involves at least one manic episode; however, some patients suffering from Bipolar Disorder experience Major Depressive episodes.

The present invention provides a method for treating the patient suffering from or susceptible to Bipolar Disorder, wherein the patient fails to experience a manic episode.

To further clarify, Applicants contemplate the treatment of both Bipolar Disorder I and Bipolar disorder II as described in the DSM-IV-R. The DSM-IV-R was prepared by the Task Force on Nomenclature and Statistics of the American Psychiatric Association, and provides clear descriptions of diagnostic categories. The skilled artisan will recognize that there are alternative nomenclatures, nosologies, and classification systems for pathologic psychological conditions and that these systems evolve with medical scientific progress.

Present therapies include Lithium, a very problematic compound, and anticonvulsants such as carbamazepine, valproic acid which induce sedation and cognitive impairments.

This method also includes the treatment of sleep disorders in all their various forms. In particular, it includes the treatment of snoring, sleep apnea, insomnia, narcolepsy, restless legs syndrome, sleep terrors, sleep walking, sleep eating, disturbances in falling asleep, and disturbance of sleep architecture.

The invention provides a method for the treatment of obsessive compulsive disorder and autism.

The invention provides a method for the treatment of cognitive disturbances related to age or MCI including Alzheimer's disease. The invention also includes the treatment of other manifestations of age or neurodegeneration-induced neural impairment such as agitation/restlessness and aggression.

Tthe present invention provides a new way to treat obsessive compulsive disorder.

In a preferred embodiment, the compounds are chosen in the group consisting of (1R,2S,4R)-(-)-2-[N,N-(dimethylaminoethoxy)]-2-phenyl-1,7,7-trimethyl-bicyclo[2.2.1]heptane, (1R,2S,4R)-(-)-2-methylaminoethoxy-2-phenyl-1,7,7-trimethyl-bicyclo[2.2.1]heptane and a pharmaceutically acceptable salt thereof. More preferably, said pharmaceutically acceptable salt is a fumarate salt.

Within the context of the invention, the term "treatment" refers to preventive, curative, palliative treatment, as well as management of patients (alleviating suffering, slowing disease progression), etc. The treatment may furthermore be conducted in combination with other agents or treatments.

As used herein, the term "mammal" or "patient" shall refer to the Mammalian class of higher vertebrates. The term "mammal" includes, but is not limited to, a human. The term "treating" as used herein includes prophylaxis of the named condition or amelioration or elimination of the condition once it has been established.

The administration may be performed by any method known to those skilled in the art, preferably by the oral route or by injection, typically by the intraperitoneal, intracerebral, intravenous, intraarterial or intramuscular route. Administration by the oral route is preferred. The administered doses may be adapted by those skilled in the art. For example, in the treatment of adult humans, dosages of from about 0.25 to 50 mg, preferably from 1 to 30 mg, and most preferably 1 to 20 mg per day may be used. A once a day dosage is normally sufficient, although divided doses may be administered. It is understood that repeated administrations may be given, possibly in combination with other active agents or any pharmaceutically acceptable vehicle.

The bicycloheptane derivatives as defined above or a pharmaceutically acceptable salt thereof will normally be administered orally or by injection and, for this purpose, it is usually employed in the form of a pharmaceutical composition.

Accordingly, pharmaceutical compositions comprising such compounds, as active ingredients associated with pharmaceutically acceptable carriers may be prepared. In making the compositions of the invention conventional techniques for the preparation of pharmaceutical compositions may be used. For example, the active ingredient will usually be mixed with a carrier, or diluted by a carrier, or enclosed within a carrier which may be in the form of a capsule, sachet, paper or other container.

When the carrier serves as a diluent, it may be solid, semi-solid or liquid material which acts as a vehicle, excipient or medium for the active ingredient. The active ingredient can be adsorbed on a granular solid container for example in a sachet. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxy-benzoate, talc, magnesium stearate or mineral oil. The compositions of the invention may, if desired, be formulated so as to provide quick, sustained or delayed release of the active ingredient after administration to the patient.

Depending on the method of administration and the therapeutic indications, the compositions may be formulated as tablets, capsules, injection solutions for parenteral use, gel or suspension for transdermal delivery, suspensions or elixirs for oral use or suppositories. Preferably the compositions are formulated in a unit dosage form, each dosage containing from 0.25 to 25 mg, more usually 1 to 20 mg, of the active ingredient. An alternative preferred composition is a unit dosage form containing from 1 to 30 mg.

The following examples are provided for purposes of illustration and are not to be construed as limiting the scope of the claimed invention.

### Examples

### Materials and methods

| **Binding Assays** | | | |
|---|---|---|---|
| **General procedures** | | | |
| Assay | Origin | Reference Cpd | Bibliography |
| D2 | Human recombinant | (+) butaclamol | Grandy et al. (1990) |
| D3 | Human recombinant | (+) butaclamol | Mackenzie et al (1994) |
| | (CHO cells) | | |
| σ | Rat cerebral cortex | haloperidol | Shirayama et al. (1993) |
| 5HT2a | Human recombinant | ketanserin | Bonhaus et al. (1995) |
| | (CHO cells) | | |
| 5HT2c | Human recombinant | mesulergine | Bonhaus et al. (1995) |
| | (CHO cells) | | |
| 5HT6 | Human | serotonin | Monsma et al (1993) |
| | Recombinant | | |
| | HEK-293 cells | | |
| Na channel | Rat cerebral cortex | veratridine | Brown (1986) |

| **Experimental conditions** | | | | | |
|---|---|---|---|---|---|
| Assay | Ligand | Conc. | Non specific | Incubation | Method of detection |
| D2 | [3H]spiperone | 0.3nM | (+) butaclamol | 60Min/22°C | Scintillation |
| | | | (10µM) | | counting |
| D3 | [3H]spiperone | 0.3nM | (+) butaclamol | 60Min/22°C | Scintillation |
| | | | (10nM) | | counting |
| σ | [3H]DTG | 8nM | haloperidol | 120Min/22°C | Scintillation |
| | | | (10µM) | | counting |
| 5HT2a | [3H]ketanserine | 0.5nM | Ketanserin | 15Min/37°C | Scintillation |
| | | | (1µM) | | counting |
| 5HT2c | [3H]mesulergine | 0.7nM | Mesulergine | 30Min/37°C | Scintillation |
| | | | (1µM) | | counting |
| 5HT6 | [3H] LSD | 2nM | Serotonin | 60Min/37°C | Scintillation |
| | | | (100µM) | | counting |
| Na | [3H]batrachotoxin | 10nM | Veratridine | 60Min/22°C | Scintillation |
| channel | | | (300µM) | | counting |

**D2 ASSAY :** Evaluation of the affinity of compounds for the human dopamine D2_{S} receptor in transfected CHO cells determined in a radioligand binding assay.
Cell membrane homogenates (5-10 µg protein) were incubated for 60 min at 22°C with 0.3 nM [³H]spiperone in the absence or presence of the test compound in a buffer containing 50 mM Tris-HCl (pH 7.4), 120 mM NaCl, 5 mM KCl, 5 mM MgCl₂ and 1 mM EDTA.
Nonspecific binding was determined in the presence of 10 µM (+)butaclamol.
Following incubation, the samples were filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters were dried then counted for radioactivity in a scintillation counter (Topcount, Packard) using a scintillation cocktail (Microscint 0, Packard).

**D3 ASSAY**: Evaluation of the affinity of compounds for the human dopamine D3 receptor in transfected CHO cells determined in a radioligand binding assay.
Cell membrane homogenates (40 µg protein) were incubated for 60 min at 22°C with 0.3 nM [³ H]spiperone in the absence or presence of the test compound in a buffer containing 50 mM Tris-HCl (pH 7.4), 120 mM NaCl, 5 mM KCl, 5 mM MgCl₂ and 1 mM EDTA.
Nonspecific binding was determined in the presence of 10 µM (+)butaclamol.
Following incubation, the samples were filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters were dried then counted for radioactivity in a scintillation counter (Topcount, Packard) using a scintillation cocktail (Microscint 0, Packard).

σ **ASSAY** : Evaluation of the affinity of compounds for the non-selective sigma receptor in the rat cerebral cortex determined in a radioligand binding assay.
Membrane homogenates of cerebral cortex (200 µg protein) were incubated for 120 min at 22°C with 8 nM [³H]DTG in the absence or presence of the test compound in a buffer containing 50 mM Tris-HCl (pH 8.0).
Nonspecific binding was determined in the presence of 10 µM haloperidol.
Following incubation, the samples were filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters were dried then counted for radioactivity in a scintillation counter (Topcount, Packard) using a scintillation cocktail (Microscint 0, Packard).

**5HT2a ASSAY :** Evaluation of the affinity of compounds for the human 5-HT_{2A} receptor in transfected HEK-293 cells determined in a radioligand binding assay.
Cell membrane homogenates (30-50 µg protein) were incubated 15 min at 37°C with 0.5 nM [3H]ketanserin in the absence or presence of the test compound in a buffer containing 50 mM Tris-HCl (pH 7.4).
Nonspecific binding was determined in the presence of 1 µM ketanserin.
Following incubation, the samples were filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters were dried then counted for radioactivity in a scintillation counter (Topcount, Packard) using a scintillation cocktail (Microscint 0, Packard).

**5HT2c ASSAY** : Evaluation of the affinity of compounds for the human 5-HT_{2C} receptor in transfected CHO cells determined in a radioligand binding assay.
Cell membrane homogenates (3-9 µg protein) were incubated for 30 min at 37°C with 3 nM [³H]mesulergine in the absence or presence of the test compound in a buffer containing 50 mM Tris-HCl (pH 7.4), 10 mM MgCl₂, 1 mM EDTA and 0.1 % BSA.
Nonspecific binding was determined in the presence of 1 µM mesulergine.
Following incubation, the samples were filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters were dried then counted for radioactivity in a scintillation counter (Topcount, Packard) using a scintillation cocktail (Microscint 0, Packard).

**5HT6 ASSAY :** Evaluation of the affinity of compounds for the human 5-HT₆ receptor in transfected HEK 293 cells determined in a radioligand binding assay.
Cell membrane homogenates (15-20 µg protein) were incubated for 60 min at 37°C with 2 nM [³H] LSD in the absence or presence of the test compound in a buffer containing 50 mM Tris-HCl (pH 7.4), 10 mM MgCl₂ and 0.5 mM EDTA.
Nonspecific binding was determined in the presence of 100 µM 5-HT.
Following incubation, the samples were filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters were dried then counted for radioactivity in a scintillation counter (Topcount, Packard) using a scintillation cocktail (Microscint 0, Packard).

**Na channel ASSAY:** Evaluation of the affinity of compounds for the Na⁺ channel (site 2) in the rat cerebral cortex determined in a radioligand binding assay.
Membrane homogenates of cerebral cortex (250 µg protein) were incubated for 60 min at 22°C with 10 nM [³H]batrachotoxinin in the absence or presence of the test compound in a buffer containing 50 mM Hepes/Tris (pH 7.4), 130 mM choline chloride, 5.4 mM KCl, 0.8 mM MgSO₄, 5.5 mM glucose, 0.15 g/l scorpion venom and 0.1 % BSA.
Nonspecific binding was determined in the presence of 300 µM veratridine.
Following incubation, the samples were filtered rapidly under vacuum through glass fiber filters (GF/B, Packard) presoaked with 0.05% BSA and rinsed several times with an ice-cold buffer containing 50 mM Hepes/Tris (pH 7.4), 130 mM choline chloride and 0.8 mM MgSO₄ using a 96-sample cell harvester (Unifilter, Packard). The filters were dried then counted for radioactivity in a scintillation counter (Topcount, Packard) using a scintillation cocktail (Microscint 0, Packard).

### Analysis and Expression of Results

The specific ligand binding to the receptor is defined as the difference between the total binding and the non specific binding determined in the presence of an excess of unlabelled ligand. The results are expressed as a percentage of control specific binding and as a percentage inhibition of control specific binding obtained in the presence of the compounds.

### Reference compounds

In each experiment, the respective reference compound was tested concurrently with the test compound in order to assess the assay suitability. The assay was rendered valid if the suitability criteria were met, in accordance with the corresponding Standard Operating Procedure.

| **target** | **Cpd A** | **Cpd B** | **Cpd C** | **Cpd D** |
|---|---|---|---|---|
| | %inhib at 1µM | %inhibition | %inhibition | %inhibition |
| D2 | 29 | 74 | 10 | 57 |
| D3 | 32 | 89 | 24 | 81 |
| *σ* | 56 | 72 | 60 | 54 |
| 5HT2a | 93 | 99 | 89 | 98 |
| 5HT2c | 79 | 99 | 65 | 97 |
| 5HT6 | 44 | 88 | 36 | 89 |
| Na channel | 50 | 65 | 58 | 78 |

Compound A is (1S,2R,4S)-(+)-2-[N,N-(dimethylaminoethoxy)]-2-phenyl-1,7,7-trimethyl-bicyclo[2.2.1]heptane, hydrochloride salt
Compound B is (1R,2S,4R)-(-)-2-[N,N-(dimethylaminoethoxy)]-2-phenyl-1,7,7-trimethyl-bicyclo[2.2.1]heptane, hydrochloride salt
Compound C is (1S,2R,4S)-(+)-2-methylaminoethoxy-2-phenyl-1,7,7-trimethyl-bicyclo[2.2.1]heptane, free base
Compound D is (1R,2S,4R)-(-)-2-methylaminoethoxy-2-phenyl-1,7,7-trimethyl-bicyclo[2.2.1]heptane, free base

### References:

Grandy et al. (1990) Proc. Natl. Acad. Sci. USA, 86, 9762-9766
Mackenzie et al (1994) Eur. J. Pharmacol. 266, 79-85
Shirayama et al. (1993) Eur. J. Pharmacol. 237, 117-126
Bonhaus et al. (1995) Brit J Pharmacol 115, 622-628
Monsma et al (1993) Mol. Pharmacol. 43, 320-327
Brown (1986) J. Neurosci &, 2064-2070

## Claims

1. Use of bicycloheptane derivatives selected in the group consisting of (1R,2S,4R)-(-)-2-[N,N-(dimethylaminoethoxy)]-2-phenyl-1,7,7-trirnethyl-bicyclo [2.2.1] heptane, (1S,2R,4S)-(+)-2-[N,N-(dimethylaminoethoxy)]-2-phenyl- 1,7,7-trimethyl-bicyclo[2.2.1]heptane, (1RS,2SR,4RS)- 2-[N,N-(dimethylaminoethoxy)]-2-phenyl-1,7,7-trimethyl-bicyclo[2.2.1]heptane, one of their active metabolites, a mixture thereof, and a pharmaceutically acceptable salt thereof for the manufacture of a medicament for therapeutic application treating autism, fibromyalgia, sleep disorders, bipolar disorder, mania, obsessive compulsive disorder, social phobia, anorexia, cognition disturbances, or age related disturbances.

2. Use according to claim 1, wherein the active metabolites are chosen in the group consisting of (1R,2S,4R)-(-)-2-methylaminoethoxy-2-phenyl-1,7,7-trimethyl-bicyclo[2.2.1]heptane, (1S,2R,4S)-(+)-2-methylaminoethoxy-2-phenyl-1,7,7-trimethyl-bicyclo[2.2.1]heptane, and (1RS,2SR,4RS)-2-methylaminoethoxy-2-phenyl-1,7,7-trimethyl-bicyclo[2.2.1]heptane.

3. Use according to claim 1, wherein said pharmaceutically acceptable acid addition salt is the fumarate salt.

4. Use according to claim 1, wherein the administration of said medicament is an oral administration.

5. Use according to any one of claim 1-4, wherein said therapeutical application is treating mania.

6. Use according to any one of claim 1-4, wherein said therapeutical application is treating bipolar disorders.

7. Use according to any one of claim 1-4, wherein said therapeutical application is treating cognition disturbance.
